# EUROPEAN PATENT APPLICATION

(11) **EP 3 895 754 A1**
(43) Date of publication of application: **20.10.2021**
(21) Application number: 19904565.9
(22) Date of filing: 26.12.2019
(51) Int. Cl.: A61M 31/00, A61B 17/28

(54) **SHEET-SHAPED OBJECT ATTACHING DEVICE**

(30) Priority: 26.12.2018 JP 2018242375
(71) Applicant: NAGASAKI UNIVERSITY, Nagasaki 852-8521 (JP); TERUMO Kabushiki Kaisha, Tokyo 151-0072 (JP)
(72) Inventor: SAKAI, Yusuke, Nagasaki-shi, Nagasaki 852-8521 (JP); EGUCHI, Susumu, Nagasaki-shi, Nagasaki 852-8521 (JP); MARUYA, Yasuhiro, Nagasaki-shi, Nagasaki 852-8521 (JP); OHASHI, Fumiya, Ashigarakami-gun, Kanagawa 259-0151 (JP); SAMESHIMA, Tadashi, Ashigarakami-gun, Kanagawa 259-0151 (JP)
(74) Representative: Prüfer & Partner mbB Patentanwälte · Rechtsanwälte
(86) International application number: PCT/JP2019/051069
(87) International publication number: WO 2020/138261

(57) **Abstract**

An object of the present invention is to provide a device for efficiently attaching a sheet-shaped object to a target site. The present invention relates to a sheet-shaped object attaching device including a support body for supporting a sheet-shaped object, and a protective member having a low coefficient of friction for protecting one surface of the support, in which the support body supporting the sheet-shaped object is rolled while being protected by the protective member and can be inserted into a tubular body.

## Description

### Technical Field

The present invention relates to a device for attaching a sheet-shaped object to a target site and a method for attaching a sheet-shaped object using the device.

### Background Art

In recent years, attempts have been made to transplant various cells for repairing damaged tissues and the like. For example, in order to repair myocardial tissues damaged by an ischemic heart disease such as angina or myocardial infarction, use of fetal cardiomyocytes, myoblast cells, mesenchymal stem cells, cardiac stem cells, ES cells, iPS cells, and the like has been attempted (Non Patent Literature 1).

As a part of such an attempt, a cell structure formed by using a scaffold and a sheet-shaped cell culture in which cells are formed in a sheet shape have been developed (Non Patent Literature 2).

Regarding a therapeutic application of the sheet-shaped cell culture, use of a cultured epidermis sheet for skin damage caused by burns and the like, use of a corneal epithelial sheet-shaped cell culture for corneal damage, use of an oral mucosal sheet-shaped cell culture for endoscopic resection of esophageal cancer, and the like have been studied, and some of these are in a stage of clinical application.

Regarding a surgical method for administering the sheet-shaped cell culture to a target site, endoscopic surgery is widely used as a minimally invasive surgical method for a human body, and various instruments for delivering the sheet-shaped cell culture to the target site for administration have been proposed.

For example, a transporting/administering instrument described in Patent Literature 1 can transport a sheet-shaped therapeutic substance up to a transplant site by forming a seat support member into a tubular shape and storing the seat support member in an outer cylinder to reduce the degree of invasion to a human body.

A sheet attaching device described in Patent Literature 2 detaches a rod-shaped member disposed along a surface of a sheet support portion by moving the rod-shaped member along the surface of the sheet support portion, and can attach the sheet-shaped substance to a target site.

A transporting/administering instrument described in Patent Literature 3 can administer a sheet using a sheet attachment/detachment means capable of applying a negative pressure for attaching and holding a sheet-shaped therapeutic substance to a sheet support and a positive pressure for detaching the sheet-shaped therapeutic substance from the sheet support.

A conveyance instrument described in Patent Literature 4 can attach a sheet-shaped therapeutic substance by ejecting a fluid from a plurality of vents formed on a surface of a head holding the sheet-shaped therapeutic substance.

### Citation List

### Patent Literature

Patent Literature 1: JP 2009-511 A
Patent Literature 2: JP 2016-187601 A
Patent Literature 3: JP 2008-173333 A
Patent Literature 4: WO 2014/069292 A

### Non Patent Literature

Non Patent Literature 1: Haraguchi et al., Stem Cells Transl Med. 2012 Feb; 1 (2): 136-41
Non Patent Literature 2: Sawa et al., Surg Today. 2012 Jan; 42 (2): 181-4

### Summary of Invention

### Technical Problem

Under these circumstances, in developing a means for efficiently attaching a sheet-shaped object to a target site, the present inventors faced a problem that it is difficult to efficiently and quickly detach a sheet-shaped object from a device without causing damage or generating wrinkles. Therefore, an object of the present invention is to solve such a problem and to achieve a rational device having a simple configuration and.

### Solution to Problem

In making intensive studies in order to solve the above problem, the present inventors have found for the first time that a sheet-shaped object can be efficiently attached to a target site while maintaining the shape of the sheet-shaped object by detaching the sheet-shaped object stepwise. As a result of further continuing studies based on such findings, the present inventors have completed the present invention.

That is, the present invention relates to the following.
[1] A sheet-shaped object attaching device including a support (a support body) for supporting a sheet-shaped object and a thread-like member engaged with (locked to) the support, in which the device can collect the support by discharging the sheet-shaped object supported by the support near a target site in a body cavity and pulling the thread-like member.
[2] The device according to [1], in which the support has at least one hole.
[3] The device according to [1] or [2], further including a protective member having a low coefficient of friction for protecting one surface of the support.
[4] The device according to [3], further including a thread-like member engaged with the protective member.
[5] A method for attaching a sheet-shaped object to a target site, the method including: a step of delivering a sheet-shaped object attaching device including a support for supporting a sheet-shaped object and a thread-like member engaged with the support to a target site; and a step of detaching the sheet-shaped object from the support and attaching the sheet-shaped object to the target site.
[6] The method according to [5], in which the support has a plurality of holes, and the sheet-shaped object is detached from the support by discharging a fluid from the plurality of holes while moving a discharge position.
[7] The method according to [5] or [6], in which the sheet-shaped object is detached from the support stepwise by pulling the thread-like member engaged with the support stepwise.
[8] A sheet-shaped object attaching device including a support for supporting a sheet-shaped object, and a protective member having a low coefficient of friction for protecting one surface of the support, in which the support supporting the sheet-shaped object is rolled while being protected by the protective member and can be inserted into a tubular body.
[9] The device according to [8], in which the support has an uneven structure on a surface supporting the sheet-shaped object.
[10] The device according to [8] or [9], in which the support has at least one hole.
[11] The device according to any one of [8] to [10], in which the sheet-shaped object, the support, and the protective member each have a circular shape.
[12] A method for attaching a sheet-shaped object to a target site, the method including: a step of rolling a support supporting a sheet-shaped object while the support is protected by a protective member, gripping the support with forceps, and inserting the support into a tubular body; a step of delivering a sheet-shaped object attaching device to a target site via the tubular body; and a step of developing the support, detaching the sheet-shaped object from the support, and attaching the sheet-shaped object to the target site.
[13] The method according to [12], in which gripping with the forceps is performed on a portion where two points where no sheet-shaped object is present are overlapped with each other.
[14] The method according to [12] or [13], in which the support is developed by releasing gripping with the forceps.
[15] The method according to any one of [12] to [14], in which the development of the support includes inverting front and back surfaces of the support.
[16] The method according to any one of [12] to [15], in which the sheet-shaped object is detached from the support stepwise by pulling an end of the support stepwise with the forceps.
[17] The method according to any one of [12] to [16], in which the support has a plurality of holes, and the sheet-shaped object is detached from the support by discharging a fluid from the plurality of holes while moving a discharge position.
[18] The method according to any one of [12] to [17], which is performed for transperitoneally inserting the sheet-shaped object into a body cavity via the tubular body and attaching the sheet-shaped object to the target site.
[19] The method according to any one of [12] to [18], in which the target site is an organ, the sheet-shaped object is a sheet-shaped cell culture for promoting wound healing of the organ, and the sheet-shaped cell culture is attached to the target site such that an extracellular matrix side of the sheet-shaped cell culture is on an organ side.

### Advantageous Effects of Invention

According to the device of the present invention, a sheet-shaped object can be efficiently attached to a target site by a simple mechanism and a simple operation. Therefore, there are large advantages in terms of operability and manufacturing cost.

In addition, the sheet-shaped object can be detached stepwise. Therefore, by detaching a part of the sheet-shaped object, positioning and attaching the part of the sheet-shaped object to a target site, and then detaching the other parts stepwise, generation of wrinkles and the like can be prevented. In particular, by detaching the sheet-shaped object with a fluid, occurrence of defects and the like can be prevented, and the sheet-shaped object can be efficiently attached while the shape of the sheet-shaped object is maintained.

### Brief Description of Drawings

Fig. 1 is a conceptual diagram of a device 1 according to a first embodiment.
Fig. 2 is a conceptual diagram of a usage example of the device 1 of Fig. 1.
Fig. 3 is a conceptual diagram illustrating one embodiment of a support 2.
Fig. 4 is a conceptual diagram of a device 1' according to a second embodiment.
Fig. 5 illustrates Example 1 using a device of the present invention.
Fig. 6 illustrates Example 2 using the device of the present invention.
Fig. 7 illustrates that postoperative perforation/peritonitis has not occurred three days after transplantation.
Fig. 8 illustrates macroscopic and histological evaluation of a site to which a sheet-shaped cell culture is attached.

### Description of Embodiments

A "sheet-shaped object" in the present invention refers to a sheet-shaped object that has low physical strength and may be torn, broken, deformed, or the like, for example, by being detached from a device. Such a sheet-shaped object is not particularly limited, but examples thereof include a sheet-shaped structure, for example, a flat membrane-like membrane tissue made of a biological material such as a sheet-shaped cell culture, and films made of various materials such as plastic, paper, woven fabric, non-woven fabric, metal, polymer, and lipid. The sheet-shaped structure may be polygonal, circular, or the like, and the width, thickness, diameter, and the like of the sheet-shaped structure may be uniform or non-uniform. As the sheet-shaped structure in the present invention, only one sheet may be used in a single layer state, or two or more sheets may be overlapped with each other to be used in a state of a laminate. In the latter case, the layers of the laminate may be connected to each other, or do not have to be connected to each other. When the layers of the laminate are connected to each other, all overlapping portions may be connected, or overlapping portions may be partially connected.

In the present invention, the "sheet-shaped cell culture" refers to an object obtained by connecting cells to each other to form a sheet. The cells may be connected to each other directly (including a case where cells are connected to each other via a cell element such as an adhesion molecule) and/or via an intervening substance. The intervening substance is not particularly limited as long as being a substance capable of connecting cells to each other at least physically (mechanically), but examples thereof include an extracellular matrix. The intervening substance is preferably derived from cells, and particularly preferably derived from cells constituting a cell culture. The cells are connected to each other at least physically (mechanically), but may be connected to each other more functionally, for example, chemically or electrically. The sheet-shaped cell culture may be constituted by one cell layer (single layer) or two or more cell layers (laminated (multilayer) body, for example, two layers, three layers, four layers, five layers, or six layers). The sheet-shaped cell culture may have a three-dimensional structure having a thickness exceeding the thickness of one cell without a clear layered structure of cells. For example, in a vertical cross section of the sheet-shaped cell culture, cells may be present in a non-uniform (for example, mosaic-like) arrangement without being uniformly aligned in the horizontal direction.

The sheet-shaped cell culture preferably contains no scaffold (support). The scaffold may be used in the present technical field in order to attach cells to a surface thereof and/or an inside thereof and maintain physical integrity of the sheet-shaped cell culture. For example, a polyvinylidene fluoride (PVDF) membrane is known. The sheet-shaped cell culture of the present invention can maintain physical integrity thereof even without such a scaffold. In addition, the sheet-shaped cell culture of the present invention preferably contains only a substance derived from cells constituting the sheet-shaped cell culture, and contains no other substance.

The cells constituting the sheet-shaped cell culture are not particularly limited as long as being able to form the sheet-shaped cell culture, and include, for example, an adherent cell (adhesive cell). The adherent cell includes, for example, an adherent somatic cell (for example, a cardiomyocyte, a fibroblast cell, an epithelial cell, an endothelial cell, a hepatocyte, a pancreatic cell, a kidney cell (a renal cell), an adrenal cell, a periodontal membrane cell, a gingival cell, a bone membrane cell, a skin cell, a synovial cell, or a chondrocyte) and a stem cell (for example, a tissue stem cell such as a myoblast or a cardiac stem cell, an embryonic stem cell, a pluripotent stem cell such as an induced pluripotent stem (iPS) cell, or a mesenchymal stem cell). The somatic cell may be a stem cell, particularly a cell differentiated from an iPS cell (iPS cell-derived adherent cell). Non-limiting examples of the cells constituting the sheet-shaped cell culture include a myoblast (for example, a myoblast cell), a mesenchymal stem cell (for example, those derived from bone marrow, an adipose tissue, peripheral blood, skin, a hair root, a muscle tissue, an endometrial membrane, placenta, and umbilical cord blood), a cardiomyocyte, a fibroblast cell, a heart stem cell, an embryonic stem cell, an iPS cell, a synovial cell, a chondrocyte, an epithelial cell (for example, an oral mucosal epithelial cell, a retinal pigment epithelial cell, or a nasal mucosal epithelial cell), an endothelial cell (for example, a vascular endothelial cell), a hepatocyte (for example, a hepatic parenchymal cell), a pancreatic cell (for example, a pancreatic islet cell), a renal cell (a kidney cell), an adrenal cell, a periodontal membrane cell, a gingival cell, a bone membrane cell, and a skin cell. Non-limiting examples of the iPS cell-derived adherent cell include a cardiomyocyte, a fibroblast cell, an epithelial cell, an endothelial cell, a hepatocyte, a pancreatic cell, a renal cell, an adrenal cell, a periodontal membrane cell, a gingival cell, a bone membrane cell, a skin cell, a synovial cell, a chondrocyte, derived from an iPS cell.

In the present invention, the "thread-like member" is not particularly limited as long as being a linear object that does not excessively damage the sheet-shaped object when being engaged with the support, and for example, any known thread or a small diameter wire rod can be used. The thread-like member may be made of a natural material (for example, silk, cotton, or metal) or a synthetic material (for example, nylon, polyester, polypropylene, or carbon fibers), and may be soft or rigid. Locking the thread-like member to the support means a state in which the thread-like member is connected to the support and can be pulled, which can be achieved, for example, by causing the thread-like member to pass through the support, or engaging the thread-like member with the support.

In the present invention, the "support" refers to a plate-like body having a flat surface capable of supporting a sheet-shaped object while maintaining the shape thereof. The support has flexibility and moderate softness, can be bent while supporting the sheet-shaped object, can be stored, for example, along an inside of a tubular body, and can be developed into a plane shape due to its resilience when being exposed from the tubular body. A material of the support is not particularly limited, and various known materials can be used singly or in combination of two or more kinds thereof. Examples of such a material include metal, a soft vinyl chloride resin, a polyurethane resin, a silicone rubber, a natural rubber, a synthetic rubber, a styrene-ethylene-butylene-styrene copolymer (SEBS), a styrene-ethylene-propylene-styrene copolymer (SEPS), an ethylene vinyl acetate copolymer (EVA), a polyamide resin such as nylon and a polyamide elastomer, a polyester resin such as polyethylene terephthalate (PET) and a polyester elastomer, an olefin-based resin such as polyethylene, a fluororubber, and a fluororesin. These can be used singly or in combination of two or more kinds thereof.

In the present invention, the "plurality of holes" means two or more through holes formed in the support. By sending a fluid from the opposite surface of the support to a surface supporting the sheet-shaped object, and discharging a fluid through the holes, the sheet-shaped object can be detached from the support surface and attached. The holes only need to be able to discharge the fluid, and it is only required to form at least one hole in the support.

The plurality of holes is preferably displaced discretely on the sheet support surface of the support such that a detachment position of the sheet-shaped object can be adjusted. For example, by forming at least one of the plurality of holes in the vicinity of an edge of the support, the sheet-shaped object can be detached from the vicinity of the edge. The arrangement of the plurality of holes can be a row × column matrix arrangement such as 2 × 2 to 40 × 40 or 5 × 5 to 20 × 20. A distance between the holes can be, for example, 0.05 mm to 10 mm or 0.5 mm to 2 mm. The diameter of each of the holes can be, for example, 0.05 mm to 10 mm or 0.3 to 0.6 mm. However, a person skilled in the art can freely select the arrangement, the distance, and the diameter without limitation to these according to the size of the sheet-shaped object, the degree of adhesion between the sheet-shaped object and the support, and the like. By making the structure of the support a mesh structure, a matrix arrangement of the plurality of holes can also be achieved.

In the present invention, the "fluid" refers to a general term for a gas and a liquid that can extrude and detach the sheet-shaped object without damaging the sheet-shaped object. The liquid is constituted by at least one kind of component, and the component is not particularly limited, but examples thereof include a liquid such as water, an aqueous solution, a non-aqueous solution, a suspension, or an emulsion. The component constituting the liquid is not particularly limited as long as having little effect on the sheet-shaped object. When the sheet-shaped object is a membrane made of a biological material, the component constituting the liquid is preferably a biocompatible substance, that is, a substance that does not cause an undesired effect such as an inflammatory reaction, an immune reaction, or a toxic reaction on living tissues and cells, or at least a substance that slightly causes such an effect.

In the present invention, the "target site" means a portion to which the sheet-shaped object is applied (attached). Examples of the target site include a site of damage (wound) in a luminal organ and the opposite side of a luminal wall corresponding to the site of damage. The "luminal organ" means an organ having a lumen housed in a body cavity, that is, an organ having a tubular or bag-like structure. Examples thereof include an organ of a gastrointestinal system, an organ of a circulatory system, an organ of a urethral system, an organ of a respiratory system, and an organ of a female reproductive system. The luminal organ is typically an organ of a gastrointestinal system. The "luminal wall" means an organ wall constituting a tube or a bag of a luminal organ. The luminal wall has an inner portion facing a lumen and an outer portion forming an outer surface of an organ. "One side of the luminal wall" means either the inside or the outside of the luminal wall. The outside with respect to the inside or the inside with respect to the outside is referred to as the "opposite side". A region located on the opposite side of the luminal wall with respect to a certain region on one side is referred to as "corresponding opposite side".

In an embodiment, in a luminal tissue with damage on at least one side of a luminal wall, by transplanting a sheet-shaped cell culture to the opposite side corresponding to a site of damage, healing of the tissue can be promoted. In particular, this is effective even against damage formed by a medical treatment such as endoscopic submucosal dissection (hereinafter referred to as ESD). Therefore, a complication caused after the medical treatment can be prevented, and prognosis can be improved.

In the present invention, "selecting a discharge position" means selecting a hole from which a fluid is discharged from among the plurality of holes. Such a selection can be performed, for example, by controlling opening and closing of the plurality of holes, and discharging a fluid while a hole at a selected specific position is opened and the other holes are closed, or discharging the fluid while a positional relationship between a position to which the fluid is delivered and the plurality of holes is changed.

In the present invention, "the sheet-shaped object is detached stepwise" means that the sheet-shaped object is detached partially and stepwise (in a stepwise fashion). For example, by partially detaching a part (edge or the like) of the sheet-shaped object, attaching the part to a target site, and then pulling the device, the sheet-shaped object can be detached while wrinkles and the like that may be generated on the sheet-shaped object are extended. In addition, by detaching the remaining part stepwise in addition to the detached part, the sheet-shaped object can be detached carefully and surely while presence or absence of wrinkles and the like that may be generated on the sheet-shaped object is checked.

By selecting a discharge position with a surface supporting the sheet-shaped object spatially separated from a target site to which the sheet-shaped object is applied, and detaching the sheet-shaped object from the support stepwise, the sheet-shaped object can be disposed while a part (edge or the like) of the sheet-shaped object is partially in contact with the target site (sheet-shaped object application site). Then, by detaching the remaining part of the sheet-shaped object stepwise, the sheet-shaped object can be disposed carefully and surely at the target site while the device is not in contact with the target site. Therefore, it is possible to prevent a disadvantage that the target site is damaged carelessly, for example.

In the present invention, the "tubular body" refers to a long pipe-shaped object having a space inside. The tubular body has openings at both ends thereof, and can deliver a fluid, the support, the thread-like member, and the like to a distal end side of the tubular body through the openings and the internal space. The tubular body is not particularly limited as long as the fluid does not leak from the tubular body, and any tubular body including a commercially available tubular body can be used. Examples of a material of the tubular body include polyethylene, polypropylene, Teflon (registered trademark), polyethylene terephthalate, polymethyl methacrylate, nylon 6,6, polyvinyl alcohol, cellulose, silicon, polystyrene, glass, polyacrylamide, polydimethyl acrylamide, and metals (for example, iron, stainless steel, aluminum, copper, and brass), but are not limited thereto.

The tubular body may be made of a flexible material.
In this case, even when a passage for insertion into a body during laparoscopic surgery, thoracoscopic surgery, or the like is bent, the tubular body can be bent according to the form of the passage, or can be bent in order to adjust a relative angle between the tubular body and a target site. The outer diameter of the tubular body is not particularly limited, but may be, for example, 2.2 to 25 mm, and preferably 5 to 15 mm. The inner diameter of the tubular body is not particularly limited, but may be, for example, 2 to 20 mm, and preferably 3 to 15 mm.

In an embodiment, the sheet-shaped cell culture of the present invention may be very fragile and difficult to handle. Therefore, the sheet-shaped cell culture of the present invention may further have a reinforcing layer for the purpose of facilitating handling and reducing a risk of breakage. The reinforcing layer may be any layer as long as being able to reinforce the structure without impairing the function of the sheet-shaped cell culture of the present disclosure, and may be, for example, a reinforcing layer containing a gel and/or a polymer. However, the reinforcing layer is to be transplanted into a living body, and therefore is preferably a biocompatible reinforcing layer containing, for example, a biocompatible gel or polymer.

The gel that can be used for the reinforcing layer of the present disclosure, preferably a biocompatible gel, may be any gel as long as not adversely affecting a living body when the gel is introduced into the living body. Examples thereof include a fibrin gel, a fibrinogen gel, a gelatin gel, and a collagen, without being limited thereto.

The polymer that can be used for the reinforcing layer of the present disclosure, preferably a biocompatible polymer, may be any polymer as long as not adversely affecting a living body when the polymer is introduced into the living body. Examples thereof include polylactic acid, polyglycolic acid, polydioxano, polyglycapro, and collagen, without being limited thereto.

As a method for forming the reinforcing layer containing a biocompatible gel, a method known in the present technical field can be used. Examples of such a method include a method for spraying a biocompatible gel or polymer, or a component as a material thereof onto a sheet-shaped cell culture, a method for laminating a sol-like biocompatible substance on a sheet-shaped cell culture and gelling the sol-like biocompatible substance, a method for soaking a sheet-shaped cell culture in a liquid gel and then solidifying the gel, and a method described in JP 2016-52271 A, without being limited thereto.

In one embodiment, the sheet-shaped cell culture of the present invention includes an extracellular matrix (ECM).

In the present invention, the extracellular matrix produced by cells constituting the sheet-shaped cell culture is formed on a culture substrate side of the sheet-shaped cell culture, for example, by seeding cells on the culture substrate, and then culturing the cells for 24 hours or more, for example, for 24 hours, 30 hours, 36 hours, 42 hours, 48 hours, 54 hours, 60 hours, 66 hours, or 72 hours, which depends on various conditions. By using a temperature-responsive substrate as the culture substrate, the sheet-shaped cell culture containing the extracellular matrix can be easily detached.

The extracellular matrix contributes to adhesion of the sheet-shaped cell culture to a transplant site, and can simplify a step of bonding the sheet-shaped cell culture to the transplant site, such as suturing, when the sheet cell culture is transplanted. in addition, the extracellular matrix is a cell-derived component, and therefore there is no disadvantage in using the extracellular matrix for transplantation together with the sheet-shaped cell culture according to the present invention.

In addition, by detaching the sheet-shaped cell culture in which the extracellular matrix is formed from the culture substrate, and spraying fibrin gel or the like onto the opposite side of the extracellular matrix to form such a reinforcing layer as described above, a sheet-shaped cell culture (laminate) in which the fibrin gel (reinforcing layer), the sheet-shaped cell culture, and the extracellular matrix are laminated in this order can be prepared.

In the present invention, the "protective member" refers to a sheet-shaped member that protects the support so as to wrap around the support when the support is moved in the tubular body. The protective member is preferably made of a material having a low coefficient of friction (for example, 0.5 or less, 0.3 or less, 0.1 or less, or 0.05 or less), and this makes it possible to smoothly slide the support on an inner surface of the tubular body while the support is protected.

### First Embodiment

Hereinafter, a device according to a preferred first embodiment of the present invention will be described in detail with reference to the drawings.

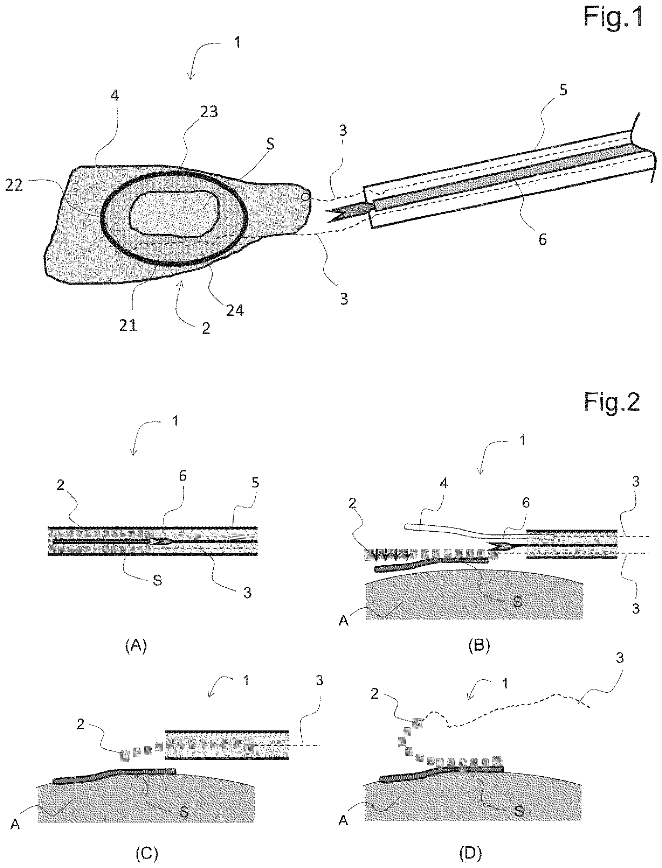

As illustrated in Fig. 1, a device 1 according to the first embodiment of the present invention includes a support 2 for supporting a sheet-shaped object S and a thread-like member 3 (illustrated by dotted lines for convenience) engaged with the support 2. The support 2 has a mesh structure 24 in which a plurality of holes 21 is formed in a matrix, and an edge 23 is formed so as to surround the mesh structure 24. The support 2 has flexibility and moderate softness, and can be bent while supporting the sheet-shaped object S and can be inserted into (stored in) a tubular body 5 or the like. By molding the support 2 such that a part of the contour of the support 2 has a protruding shape, bringing a top of the protruding portion into contact with an opening of the tubular body 5, and pressing the top against the opening to bend the support 2, the support 2 can be easily pushed into the tubular body 5 or the like. When the support 2 is pushed (or pulled) out of the tubular body 5, the support 2 can be developed into a plane shape due to its resilience.

At the edge 23 of the support 2, a locking portion 22 for locking the thread-like member 3 is disposed. By pulling the thread-like member 3 engaged with the support 2, the support 2 can be moved, pulled out of the tubular body 5, or pulled into the tubular body 5. The locking portions 22 may be disposed at a plurality of locations on the edge 23, but the plurality of holes 21 may be used as the locking portions 22. A plurality of the thread-like members 3 may be engaged with the support 2. By gripping and moving the support 2 with forceps 6 and the like, the support 2 can be pushed into the tubular body 5 or can be pushed out of the tubular body 5. In addition, by protecting one surface of the support 2 with a protective member 4 having a low coefficient of friction, and disposing the support 2 such that the protective member 4 slides on an inner surface of the tubular body 5, the support 2 can be easily inserted. That is, the device 1 can further include at least one of the protective member 4, the tubular body 5, and the forceps 6.

As illustrated in Fig. 2, when the device 1 is used, the support 2 supporting the sheet-shaped object S is inserted into the tubular body 5 (Fig. 2A). Next, the support 2 is disposed near a target site A and the sheet-shaped object S is detached (Fig. 2B). Then, the support 2 is collected with the thread-like member 3 (Fig. 2C). The sheet-shaped object S can also be detached by placing the support 2 supporting the sheet-shaped object S at the target site A and removing the support 2 (Fig. 2D).

As illustrated in Fig. 2A, the sheet-shaped object S is disposed on the support 2 with a sticky liquid such as water interposed between the sheet-shaped object S and the support 2, and the support 2 is bent (rolled) and inserted into (stored in) the tubular body 5. At this time, by disposing the sheet-shaped object S inside the support 2, the sheet-shaped object S can be protected. Instead of the tubular body 5, a port used in endoscopic surgery or the like may be used. Furthermore, by disposing the support 2 inside the protective member 4 (not illustrated), the support 2 can be easily moved in the tubular body 5 by utilizing the low coefficient of friction of the protective member 4. In this case, by locking the thread-like member 3 to the protective member 4, the protective member 4 can be easily collected. As the protective member 4, a nylon mesh having a diameter of 80 µm or the like can be used.

When the support 2 is bent (rolled) and inserted into the tubular body 5, the support 2 may be inserted by rolling the support 2 by hand, the support 2 may be bent and inserted by pulling the support 2 with the thread-like member 3 (not illustrated), bringing the support 2 into contact with an opening of the tubular body 5, and pressing the support 2 against the opening of the tubular body 5, or the support 2 may be bent and inserted by gripping the support 2 with the forceps 6 and pressing the support 2 against the opening of the tubular body 5. At this time, by pressing a top of the protruding portion of the support 2 against the tubular body 5, the support 2 can be bent more easily. When the protective member 4 is used, by molding the protective member 4 such that a part of the contour of the protective member 4 has a protruding shape, and pressing a top of the protruding portion against the tubular body 5, the support 2 can be bent together with the protective member 4.

When the tubular body 5 has already been inserted into a body cavity, by inserting the device 1 from a proximal end side of the tubular body 5 and pushing out the device 1 to a distal end side of the tubular body 5, the device 1 can be delivered into the body cavity. When a distal end side of the tubular body 5 is outside the body cavity, by inserting the device 1 from the distal end side of the tubular body 5 and storing the device 1 therein, then inserting the tubular body 5 into the body cavity, and pushing out the device 1 to the distal end side, the device 1 can be delivered into the body cavity. In the latter case, a movement distance of the device 1 is short, and therefore the procedure is simple. In addition, due to long distance movement of the support 2 within the tubular body 5, a disadvantage such as careless disengagement of the support 2 from the forceps 6 can be reduced.

As illustrated in Fig. 2B, when the sheet-shaped object S is attached to the target site A, a distal end of the tubular body 5 in which the support 2 is stored is placed close to the target site A for positioning. At this time, by providing a certain distance between the tubular body 5 and the target site A, for example, a disadvantage that the tubular body 5 and the support 2 damage the target site A can be prevented. Next, the support 2 is gripped with the forceps 6 and the like, pushed out toward a distal end side of the tubular body 5, and the support 2 is developed into a plane shape. At this time, when the protective member 4 is used, by locking the thread-like member 3 to the protective member 4 and pulling the thread-like member 3, the protective member 4 can be removed from the support 2 and can be collected in the tubular body 5. In addition, by using forceps (not illustrated) different from the forceps 6 inserted into the tubular body 5, the support 2 may be gripped, or the protective member 4 may be collected.

Then, by spraying a fluid from the surface of the support 2 opposite to the surface supporting the sheet-shaped object S, the fluid is discharged from the plurality of holes 21 in the direction of the arrow to detach the sheet-shaped object S from the support 2. At this time, by moving the position onto which the fluid is sprayed (fluid discharge position) from one end of the support 2 to the other end thereof, the sheet-shaped object S can be detached stepwise. For example, by partially detaching a part of the sheet-shaped object S (for example, an edge), attaching the part to a target site, and detaching the remaining part stepwise, the sheet-shaped object S can be detached carefully and surely and can be attached to the target site A while presence or absence of wrinkles and the like that may be generated on the sheet-shaped object S is checked.

In Fig. 2B, the support 2 holds a certain distance from the target site by being gripped with the forceps 6, but this can also be achieved by using the thread-like member 3 instead of the forceps 6. That is, by disposing a plurality of (two or more) locking portions in the support 2 to lock the plurality of thread-like members 3 and pulling the plurality of thread-like member 3, the detachment operation can be performed in a state where the support 2 is floated in the air, that is, in a state where a certain distance from the target site is maintained. For example, when there are two thread-like members 3, by pulling the two thread-like members 3 in opposite directions, the support 2 can be floated in the air. When there are three or more thread-like members 3, by pulling the three or more thread-like members 3 radially, the support 2 can be floated in the air. Therefore, at least the edge 23 of the support 2 preferably has enough strength to withstand a tensile stress caused by the thread-like member 3.

As illustrated in Fig. 2C, after the sheet-shaped object S is attached to the target site A, by pulling the thread-like member 3 engaged with the support 2 into the tubular body 5, the support 2 can be collected. Also at this time, the support 2 is pressed against the opening of the tubular body 5, rolled, and stored therein. Therefore, the contour of the support 2 can also be molded such that both a side pushed into the tubular body 5 and a side pulled into the tubular body 5 have protruding shapes (for example, a circular shape as illustrated by the support 2 in Fig. 1). By collecting (storing) the support 2 in the tubular body 5, the support 2 can be taken out of the body cavity simply by pulling out the tubular body 5, and for example, a disadvantage that the developed support 2 damages another part in the body cavity is unlikely to occur. In addition, even when the support 2 falls off at the time of collection, the support 2 can be collected by pulling the thread-like member 3. In this way, by preferably using the device 1 including the support 2 for supporting the sheet-shaped object S and the thread-like member 3 engaged with the support 2 in combination with the protective member 4, the tubular body 5, the forceps 6, and the like, the sheet-shaped object S can be efficiently attached.

As illustrated in Fig. 2D, by placing the support 2 supporting the sheet-shaped object S at the target site A, that is, by attaching the sheet-shaped object S to the target site A together with the support 2 and pulling the thread-like member 3 engaged with the support 2 stepwise, the support 2 can be removed from the sheet-shaped object S stepwise, that is, the sheet-shaped object S can be detached (discharged) from the support 2 stepwise and attached to the target site A. By adjusting a direction in which the thread-like member 3 is pulled, a speed at which the thread-like member 3 is pulled, an angle at which the thread-like member 3 is pulled, and the like, the device 1 of the present invention can adjust a detachment (discharge) position of the sheet-shaped object S and a speed thereof, and therefore can detach the sheet-shaped object carefully and surely while presence or absence of wrinkles and the like that may be generated on the sheet-shaped object is checked. In this case, by pulling the thread-like member 3 engaged with a distal end side (left side in the drawing) of the support 2 to a proximal end side (right side in the drawing) of the support 2, and removing the support 2 such that a bending angle of the support 2 is acute, the sheet-shaped object S can be prevented from following the support 2. In addition, by removing the support 2 from the sheet-shaped object S while discharging a fluid from the plurality of holes 21, the sheet-shaped object S can be prevented from following the support 2. However, since this function can be achieved by pulling the thread-like member 3, the plurality of holes 21 of the support 2 can be omitted.

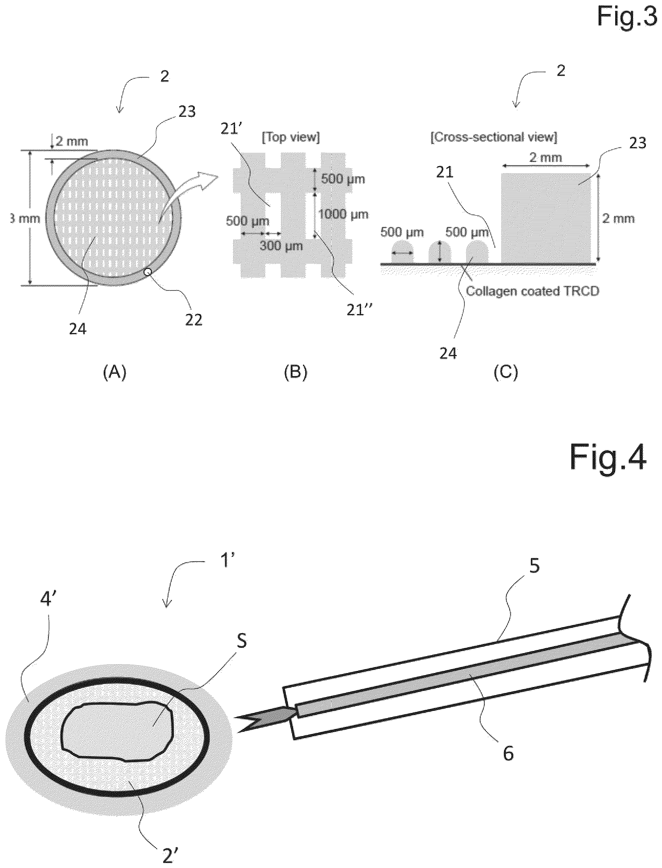

Figs. 3A to 3C illustrate one embodiment of the support 2. As illustrated in the drawings, the support 2 has the mesh structure 24 in which the plurality of holes 21 is displaced in a matrix, and the edge 23 is formed so as to surround the mesh structure (Fig. 3A). As illustrated in Fig. 3B, holes 21' and 21" each have a rectangular shape of 300 µm × 1000 µm, and a distance between the holes 21' and the hole 21" is 500 µm. As illustrated in Fig. 3C, the size of the edge 23 of the support 2 is 2 mm × 500 µm, and the size of a grid portion of the mesh structure 24 of the support 2 is 500 µm × 500 µm. As illustrated, the edge 23 is thicker than the mesh structure 24. Therefore, for example, by inserting the thread-like member 3 into the hole 21 between the edge 23 and the mesh structure 24, the hole 21 can also be used as the locking portion 22. That is, when the thread-like member 3 is inserted into the hole 21 and used as the locking portion 22, and the thread-like member 3 is pulled in the horizontal direction of the support 2, the thread-like member 3 is caught by the edge 23, and therefore, for example, a disadvantage that the support 2 is broken is unlikely to occur. In addition, the support 2 has an uneven (concavity-and-convexity) surface because mesh portions of the mesh structure 24 and the plurality of holes 21 are alternately displaced, therefore can exhibit a frictional force for preventing the sheet-shaped object S from being displaced horizontally with respect to the support 2, has a small contact area, and therefore can be easily detached from the sheet-shaped object S. Therefore, when the plurality of holes 21 of the support 2 is omitted as described above, by molding the support 2 so as to have an uneven surface, the frictional force can be exhibited.

As is clear from the above, the device 1 only needs to include at least the support 2 for supporting the sheet-shaped object S and the thread-like member 3 engaged with the support 2. In addition, by placing the support 2 supporting the sheet-shaped object S at the target site A, and removing the support 2 stepwise while pulling the support 2 with the thread-like member 3 as described above, as described in Fig. 2B, the sheet-shaped object S can be detached stepwise, that is, the sheet-shaped object can be detached carefully and surely while presence or absence of wrinkles and the like that may be generated on the sheet-shaped object is checked. The support 2 can also be collected using a laparoscopic surgical excision bag (such as INZII (registered trademark)).

As described above, according to the device 1 according to the first embodiment of the present invention, a sheet-shaped object can be efficiently attached to a target site by a simple mechanism and a simple operation. Therefore, there are large advantages in terms of operability and manufacturing cost.

In addition, the sheet-shaped object can be detached stepwise. Therefore, by detaching a part of the sheet-shaped object, positioning and attaching the part of the sheet-shaped object to a target site, and then detaching the other parts stepwise, generation of wrinkles and the like can be prevented. In particular, by detaching the sheet-shaped object with a fluid, occurrence of defects and the like can be prevented, and the sheet-shaped object can be efficiently attached while the shape of the sheet-shaped object is maintained.

The device of the present invention can be used sequentially, for example, through the following steps.
(1) The support on which the sheet-shaped object is disposed is brought close to a target site.
(2) A fluid is discharged from some of the plurality of holes.
(3) A detached portion is attached to the target site.
(4) A discharge position is moved, and the fluid is discharged from another hole.
(5) The detached portion is attached to the target site.

In order to dispose the support on which the sheet-shaped object is disposed in a state of being spatially separated from the target site, the support on which the sheet-shaped object is disposed only needs to be brought close to the target site. At this time, the sheet-shaped object can be floated in the air using the thread-like member so as not to come into contact with the target site. Even after the sheet-shaped object is detached from the sheet support, the state in which the sheet-shaped object is not in contact with the target site can be maintained until the sheet-shaped object is attached to the target site. Detachment and attachment of the sheet-shaped object may be performed separately or simultaneously. In addition, after the sheet-shaped object is attached, a step of spraying a fluid onto the attached sheet-shaped object may be added. As a result, the sheet-shaped object can be more surely bonded to the target site. At this time as well, the sheet-shaped object can be bonded to the target site in a state where the sheet support is not in contact with the target site. In addition, by spraying a gel and/or a polymer as a fluid, a reinforcing layer that reinforces the sheet-shaped object may be formed.

Hereinabove, the present invention has been described with reference to the illustrated embodiment, but the present invention is not limited thereto.

In the present invention, each component can be replaced with any component capable of exhibiting a similar function, or any component can be added.

For example, as described above, the support 2 can be collected by pulling the thread-like member 3, but the thread-like member 3 can be omitted by performing such collection with the forceps 6. Hereinafter, a device of the present invention in which the thread-like member 3 is omitted will be described.

### Second Embodiment

Hereinafter, a device according to a preferred second embodiment of the present invention will be described in detail with reference to the drawings. In the present embodiment, description will be made by assuming that the device does not include the thread-like member 3 and that the sheet-shaped object S is a sheet-shaped cell culture (laminate) obtained by laminating a fibrin gel (reinforcing layer), the sheet-shaped cell culture, and an extracellular matrix in this order.

As illustrated in Fig. 4, a device 1' according to the second embodiment of the present invention includes a support 2' for supporting the sheet-shaped object S and a protective member 4' having a low coefficient of friction for protecting one surface of the support 2'. In the present embodiment, the support 2' is molded so as to have an uneven surface, and can exhibit a frictional force for preventing the sheet-shaped object S from being displaced in the horizontal direction with respect to the support 2'. In addition, a contact area between the support 2' and the sheet-shaped object S is small due to the uneven shape, and therefore the sheet-shaped object S can be easily detached from the support 2'.

The support 2' and the protective member 4' each have a circular shape. That is, the sheet-shaped object S (sheet-shaped cell culture) generally has a circular shape, and therefore can be appropriately protected by the support 2'. In addition, the protective member 4' does not have an unnecessary area, and therefore friction of the protective member 4' can be minimized when the protective member 4' slides on an inner surface of the tubular body 5. In addition, by forming each of the support 2' and the protective member 4' in a circular shape, as described above, simply by bringing a circular top (protruding portion) into contact with an opening of the tubular body 5 and pressing the top against the opening, the device 1' can be easily bent and pushed into the tubular body 5.

When the sheet-shaped object S is attached to a target site A (not illustrated), first, the sheet-shaped cell culture (laminate) is placed on the support 2' such that an extracellular matrix side faces up. A surface of the support 2' on the opposite side to the side on which the sheet-shaped object S is placed is covered with the protective member 4'. The device 1' is rolled such that the sheet-shaped object S is inside. Two points where no sheet-shaped object S is present (for example, both ends of the support 2' or both ends of the protective member 4') are overlapped with each other. Next, the overlapping portion is pressed (gripped) by the forceps 6 and inserted into the tubular body 5 (for example, a laparoscopic port). At this time, a distal end side (tubular body 5 side) of the rolled device 1' is thin like a distal end of a needle (due to its circular shape), and therefore the distal end side of the rolled device 1' can be easily inserted into the tubular body 5. When the tubular body 5 is moved to the target site A (for example, an organ), the device 1' is pushed out of the tubular body 5, and pressing by the forceps 6 is released, as described above, the support 2' is developed into a plane shape due to its resilience. Therefore, the sheet-shaped object S can be appropriately delivered to the target site A without generating wrinkles and the like.

Next, front and back surfaces of the support 2' are inverted (turned over) such that the sheet-shaped cell culture faces the target site A side, that is, such that an extracellular matrix (ECM) side of the sheet-shaped cell culture faces the target site A side. The device 1' (support 2' and protective member 4') supporting the sheet-shaped object S is placed at the target site A. Then, the protective member 4' and the support 2' are removed in this order using the forceps 6 (or other forceps). At this time, by gripping an end of the support 2' with the forceps 6 and pulling the end stepwise, the sheet-shaped object S can be detached stepwise as described in Fig. 2D, that is, the sheet-shaped object S can be detached carefully and surely while presence or absence of wrinkles and the like that may be generated on the sheet-shaped object S is checked. In addition, at this time, by discharging a fluid from a plurality of holes while moving a discharge position, the sheet-shaped object S can be surely detached from the support 2' and attached onto the target site A.

In addition, due to the uneven shape of the support 2', the sheet-shaped object S can be easily detached from the support 2'.

The support 2' and/or the protective member 4' can also be collected in the tubular body 5. At this time, the support 2' and the protective member 4' each have a circular shape, and therefore can be collected in the tubular body 5 by gripping any portion of an end of the circular shape with the forceps 6. That is, any portion of the end of the circular shape grips the top of such a protruding portion as described above. By pressing this top against the tubular body 5, the support 2' and the protective member 4' can be easily bent and collected. Therefore, the collection operation can be performed efficiently without precisely adjusting the orientations of the tubular body 5 and the forceps 6, positioning of the forceps 6 with respect to the support 2' and/or the protective member 4', and the like.

As described above, according to the device 1' according to the second embodiment of the present invention, a sheet-shaped object can be efficiently attached to a target site by a simple mechanism and a simple operation. Therefore, there are large advantages in terms of operability and manufacturing cost.

In addition, the sheet-shaped object can be detached stepwise. Therefore, by detaching a part of the sheet-shaped object, positioning and attaching the part of the sheet-shaped object to a target site, and then detaching the other parts stepwise, generation of wrinkles and the like can be prevented. In particular, by detaching the sheet-shaped object with a fluid, occurrence of defects and the like can be prevented, and the sheet-shaped object can be efficiently attached while the shape of the sheet-shaped object is maintained.

Hereinabove, the present invention has been described with reference to the illustrated embodiment, but the present invention is not limited thereto.

In the present invention, each component can be replaced with any component capable of exhibiting a similar function, or any component can be added.

### Example 1: Transplantation of sheet-shaped cell culture into organ

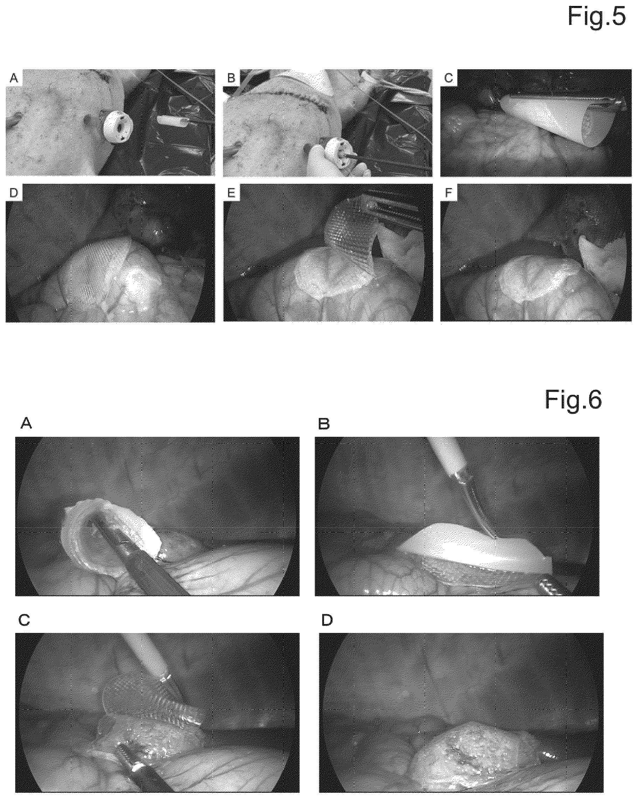

A sheet-shaped cell culture obtained by laminating a fibrin gel, the sheet-shaped cell culture, and an ECM in this order from the top was placed on a PDMS support with a plurality of holes to which a thread-like member was locked such that an ECM side faced up. The side opposite to the side on which the support was placed was covered with a nylon mesh protective member. The protected sheet-shaped cell culture was gripped with laparoscopic gripping forceps while being rolled such that the sheet-shaped cell culture was inside, and was brought close to a laparoscopic port inserted into a body cavity (Fig. 5A). Then, the rolled and protected sheet-shaped cell culture was inserted through an opening (diameter 12 mm) of the laparoscopic port, and was caused to reach an abdominal cavity (Fig. 5B). Next, the sheet-shaped cell culture was brought close to a target organ (Fig. 5C). Gripping with the gripping forceps was released, and the sheet-shaped cell culture was developed. Front and back surfaces of the support were inverted. An ECM side of the sheet-shaped cell culture was disposed on the target organ (Fig. 5D). Then, the protective member was removed, the support was removed (Fig. 5E), and only the sheet-shaped cell culture was attached to the target organ (Fig. 5F). By pulling the thread-like member stepwise when the support was removed, the sheet-shaped cell culture could be appropriately detached. In addition, by pulling the thread-like member, the support and the protective member could be appropriately collected. Delivery (transportation) of the sheet-shaped cell culture, attachment thereof to the target organ, and detachment of the support were each performed 10 times, and all were successful.

### Example 2: Transplantation of sheet-shaped cell culture into duodenum for porcine duodenal ESD model

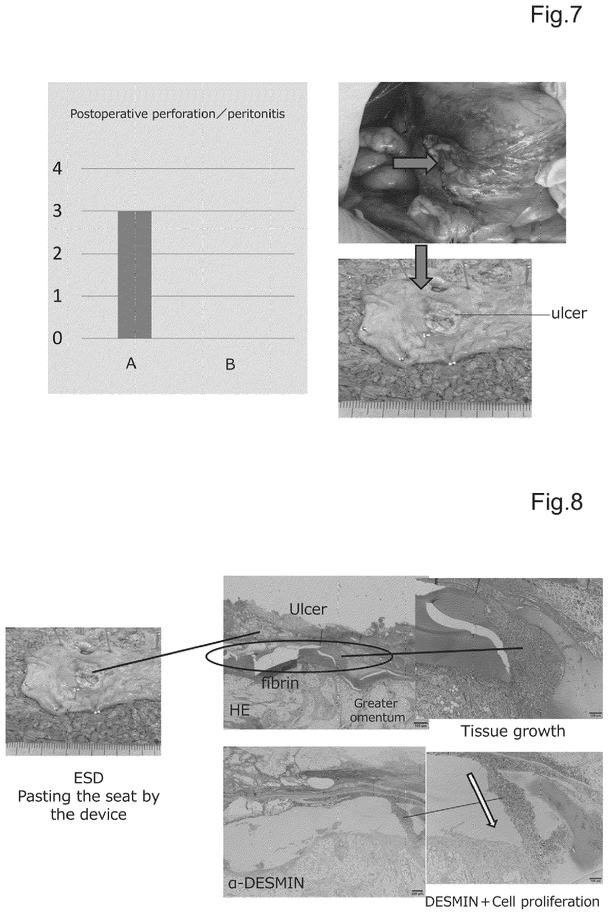

For a porcine duodenal ESD model with 100% delayed perforation after ESD, an autologous myoblast cell sheet was transplanted laparoscopically on a serosal side of the duodenum using the device, and effectiveness was evaluated. A sheet-shaped cell culture obtained by laminating a fibrin gel, the sheet-shaped cell culture, and an ECM in this order from the top was placed on a silicone film (support) such that an ECM side faced up. The side opposite to the side on which the support was placed was covered with a mesh (protective member). The protected sheet-shaped cell culture was rolled such that the sheet-shaped cell culture was inside. A portion on which the sheet-shaped cell culture was not placed was pressed by forceps, and was brought close to a laparoscopic port inserted into a body cavity. Then, the rolled and protected sheet-shaped cell culture was inserted through an opening (diameter 12 mm) of the laparoscopic port, and was caused to reach an abdominal cavity. Next, the sheet-shaped cell culture was brought close to a target site (affected portion) in the duodenum (Fig. 6A). Gripping with the gripping forceps was released, and the sheet-shaped cell culture was developed. Front and back surfaces of the support were inverted such that an ECM side of the sheet-shaped cell culture was bonded to the target site. Thereafter, the mesh (Fig. 6B) was detached, and the silicone film (Fig. 6C) was detached. Then, only the sheet-shaped cell culture was disposed (attached) onto the target site (Fig. 6D). When the support was detached, the silicone film and the sheet-shaped cell culture could be appropriately detached using gripping forceps. Then, the protective member and the support were gripped with forceps and stored (collected) in the laparoscopic port again, and it was confirmed that only the sheet-shaped cell culture could be attached to the target site. As a result, a success ratio of transporting, attaching, and detaching the sheet-shaped cell culture with this device was 100%. In addition, it was confirmed that postoperative perforation/peritonitis had not occurred three days after cell sheet transplantation (A: not occurred, B: occurred) (*perforation was performed during ESD in one case) (Fig. 7). Thereafter, the specimen was extracted, and the site to which the sheet-shaped cell culture was attached was evaluated macroscopically and histologically. Proliferation of tissues at the transplant site was confirmed, and the residual sheet-shaped cell culture was confirmed (Fig. 8: histopathological findings (duodenum)).

### Reference Signs List

- 1, 1': Device
- 2, 2': Support body
- 21, 21', 21": Hole
- 22: Locking portion
- 23: Edge
- 24: Mesh structure
- 3: Thread-like member
- 4, 4': Protective member
- 5: Tubular body
- 6: Forceps
- S: Sheet-shaped object
- A: Target site

## Claims

1. A sheet-shaped object attaching device comprising:
a support body supporting a sheet-shaped object; and a thread-like member engaged with the support body, wherein
the device can release the sheet-shaped object supported by the support body near a target site in a body cavity, and collect the support body by pulling the thread-like member.

2. The device according to claim 1, wherein the support body has at least one hole.

3. The device according to claim 1 or 2, further comprising a protective member having a low coefficient of friction for protecting one surface of the support.

4. The device according to claim 3, further comprising a thread-like member engaged with the protective member.

5. A method for attaching a sheet-shaped object to a target site, the method comprising:
delivering a sheet-shaped object attaching device including a support body supporting a sheet-shaped object and a thread-like member engaged with the support body to a target site; and
detaching the sheet-shaped object from the support body and attaching the sheet-shaped object to the target site.

6. The method according to claim 5, wherein the support body has a plurality of holes, and the sheet-shaped object is detached from the support body by discharging a fluid from the plurality of holes while moving a position of the dischargeing.

7. The method according to claim 5 or 6, wherein the sheet-shaped object is detached from the support body in a stepwise fashion by pulling the thread-like member engaged with the support in a stepwise fashion.

8. A sheet-shaped object attaching device comprising:
a support body supporting a sheet-shaped object; and a protective member having a low coefficient of friction for protecting one surface of the support, wherein
the support body supporting the sheet-shaped object is rolled while being protected by the protective member and can be inserted into a tubular body.

9. The device according to claim 8, wherein the support body has an uneven structure on a surface supporting the sheet-shaped object.

10. The device according to claim 8 or 9, wherein the support body has at least one hole.

11. The device according to any one of claims 8 to 10, wherein the sheet-shaped object, the support body, and the protective member each have a circular shape.

12. A method for attaching a sheet-shaped object to a target site, the method comprising:
rolling a support body supporting a sheet-shaped object while the support body is protected by a protective member, gripping the support body with forceps, and inserting the support body into a tubular body;
delivering a sheet-shaped object attaching device to a target site via the tubular body; and
developing the support body, detaching the sheet-shaped object from the support body, and attaching the sheet-shaped object to the target site.

13. The method according to claim 12, wherein gripping with the forceps is performed on a portion where two points where no sheet-shaped object is present are overlapped with each other.

14. The method according to claim 12 or 13, wherein the support body is developed by releasing gripping with the forceps.

15. The method according to any one of claims 12 to 14, wherein the development of the support includes inverting front and back surfaces of the support.

16. The method according to any one of claims 12 to 15, wherein the sheet-shaped object is detached from the support body in a stepwise fashion by pulling an end of the support body in a stepwise fashion with the forceps.

17. The method according to any one of claims 12 to 16, wherein the support body has a plurality of holes, and the sheet-shaped object is detached from the support body by discharging a fluid from the plurality of holes while moving a discharge position.

18. The method according to any one of claims 12 to 17, which is performed for transperitoneally inserting the sheet-shaped object into a body cavity via the tubular body and attaching the sheet-shaped object to the target site.

19. The method according to any one of claims 12 to 18, wherein the target site is an organ, the sheet-shaped object is a sheet-shaped cell culture for promoting wound healing of the organ, and the sheet-shaped cell culture is attached to the target site such that an extracellular matrix side of the sheet-shaped cell culture is on an organ side.
